# FASCICULE DE BREVET EUROPEEN

(11) **EP 0 916 951 B1**
(45) Date de publication et mention de la délivrance du brevet: **02.01.2003**
(21) Numéro de dépôt: 98430022.8
(22) Date de dépôt: 17.09.1998
(51) Int. Cl.: G01N 33/543

(54) **Procédé d'étalonnage d'un système de dosage de type ligand - anti ligand**
Eichmethode für Dosiersysteme vom Typ Ligand-Antiligand
Calibration method for ligand-antiligand test system

(30) Priorité: 18.09.1997 FR 9711848
(43) Date de publication de la demande: 19.05.1999
(73) Titulaire: Immunotech, 13276 Marseille Cédex 9 (FR)
(72) Inventeur: Fert, Vincent, 13190 Allauch (FR); Delaage, Michel, 13001 Marseille (FR); Camilla, Christophe, 13009 Marseille (FR); Defoort, Jean-Philippe, 13009 Marseille (FR); Prato, Sabine, 13006 Marseille (FR)
(74) Mandataire: Rinuy, Santarelli

(56) Documents cités:
- EP-A- 0 126 450
- EP-A- 0 296 136
- WO-A-91/00509

## Description

La présente invention concerne un nouveau procédé d'étalonnage interne du dosage d'analytes dans un échantillon, ainsi que les trousses de réactifs pour les mettre en oeuvre.

Il existe actuellement trois procédés, qui permettent l'étalonnage des dosages effectués dans les laboratoires.

Le premier, utilisé dans le cadre de dosages de type manuel aussi bien qu'automatique, repose sur l'utilisation de solutions étalons qui subissent le même traitement que les échantillons inconnus. A l'issue de la réaction, les signaux mesurés correspondant aux solutions étalons sont exploités pour tracer une courbe d'étalonnage dont l'équation est une fonction d'interpolation calculée par ajustement de paramètres. Les valeurs numériques des signaux correspondant aux échantillons inconnus, obtenues dans la même session de dosage, sont introduites dans l'équation de la courbe d'étalonnage qui donne alors les concentrations inconnues. Dans la pratique, cet étalonnage oblige l'utilisateur à traiter les solutions étalons à chaque session de dosage, ce qui augmente le coût des séries courtes et entraîne un risque de dérive pour les séries longues.

Un deuxième procédé d'étalonnage, utilisé dans le cadre de dosages automatisés seulement, repose sur l'utilisation d'une courbe standard sous forme d'équation mémorisée, obtenue lors de la fabrication du lot de réactifs, avec des solutions étalons, dans le laboratoire du fabricant. L'équation mémorisée est affectée à un lot de réactifs et a une validité n'excédant pas une période déterminée. En outre, l'équation mémorisée peut-être modifiée dans le laboratoire de l'utilisateur, grâce à des solutions étalons fournies dans la trousse de dosage.

Ce dernier procédé permet de s'affranchir de la réalisation d'une courbe d'étalonnage à chaque session de dosage et améliore donc significativement la pratique des dosages en réduisant le temps de mise en oeuvre ainsi que le coût en réactifs.

Cependant, cette dernière méthode est mise en pratique uniquement dans le cadre d'une automatisation poussée, car Il faut que les conditions des dosages, dans le laboratoire de l'utilisateur, soient strictement identiques à celles ayant conduit à la détermination de l'équation de la courbe d'étalonnage mémorisé dans le laboratoire du fabricant. Dans un automate, les temps d'incubation et d'agitation sont facilement contrôlés. Par contre, d'autres paramètres tels que la conservation des réactifs, la température de l'incubation ou la qualité du système de détection (les photomultiplicateurs par exemple) peuvent agir de façon aléatoire sur le déroulement du dosage, d'un laboratoire à l'autre ou d'un instrument à l'autre, et peuvent donc introduire une imprécision dans la méthode d'étalonnage par courbe mémorisée. D'autre part, le ré-étalonnage de la courbe mémorisé nécessite de procéder à une session de calibration distincte par analyte.

Le troisième procédé est utilisé dans le cadre de systèmes de détection semi-quantitatifs. Une première surface, portant les réactifs spécifiques qui permettent la détection du composé recherché, est adjacente à une deuxième surface qui comporte des réactifs permettant la révélation d'un signal de même nature que le signal spécifique. Les deux surfaces sont mises en contact, simultanément avec le même échantillon biologique. Ce signal adjacent est utilisé comme contrôle de la réaction de révélation ou comme valeur seuil pour indiquer, par comparaison si l'échantillon est positif ou négatif. Le brevet européen n° 0093613 décrit un tel procédé. Un autre procédé similaire figure dans la demande de brevet européen N° 0 253 464. Une zone test est juxtaposée à la surface d'analyse et peut servir à calibrer le dosage lorsque la réaction est strictement linéaire par rapport aux concentrations. Il faut noter que dans ces deux procédés, la détection du signal contrôle n'est pas simultanée à celle du signal de mesure et donc sujette à une incertitude supplémentaire due aux fluctuations du détecteur.

Il est donc souhaitable de simplifier l'étalonnage des dosages de type manuel et d'améliorer significativement l'étalonnage des dosages automatisés et semi-quantitatifs.

En particulier, il est souhaitable de permettre l'étalonnage d'un dosage lors de la mise en oeuvre dudit dosage sur l'échantillon étudié, sans avoir recours à des manipulations supplémentaires d'étalonnage des réactifs lors de la session d'expérimentation.

Il est tout aussi souhaitable que l'étalonnage prenne en compte les effets de matrice, qui varient d'un échantillon à l'autre, comme les augmentations ou diminutions de fixation ou les fixations non spécifiques des réactifs de révélation, ou encore les perturbations dues en particulier aux écarts de temps d'incubation des différents réactifs en présence ou à l'agitation du milieu réactionnel. Il est encore souhaitable que le procédé compense non seulement les écarts des systèmes de détection d'un instrument à l'autre mais aussi les fluctuations rapides d'un même instrument. Il est enfin souhaitable d'étalonner simultanément plusieurs dosages d'analytes différents.

C'est pourquoi la présente invention a pour objet un nouveau procédé d'étalonnage d'un système de dosage d'un analyte dans un échantillon biologique mettant en jeu une interaction spécifique de type ligand/anti-ligand telle qu'une interaction antigène/anticorps ou enzyme/inhibiteur ou encore acide nucléique/acide nucléique, dans un schéma réactionnel de type «sandwich» ou «compétition», caractérisé en ce que l'on fait réagir un échantillon biologique renfermant le cas échéant l'analyte avec,
- une première catégorie de microsphères appelées microsphères de calibration caractérisable par la taille, le spectre de fluorescence (F1) ou par l'intensité de sa fluorescence (F1), ces microsphères comportant un site ligand E1, et de préférence formé de plusieurs sous-catégories se distinguant les unes des autres par la quantité de sites ligands E1, et avec,
- un traceur formé par un anti-ligand spécifique du site ligand E₁ et couplé à un composé fluorophore F2 d'une fluorescence différente de celle (F1) qui peut caractériser les microsphères de calibration et avec
- une seconde catégorie de microsphères, appelées microsphères de dosages, différente de la première catégorie de microsphères sur le plan ou de la taille, ou du spectre de fluorescence (F1) ou de l'intensité de la fluorescence (F1), et qui constitue le support de la réaction analytique, selon l'une des modalités de mise en oeuvre suivantes:
   i) dans le cas où l'analyte comprend un site E1 identique à celui porté par les microsphères de calibration, ladite seconde catégorie est couplée à un anti-ligand spécifique d'un site ligand E2 de l'analyte, et l'on utilise aussi dans la réaction un traceur anti-ligand E1 couplé au composé fluorophore F2 (calibration homologue),
   ii) ladite seconde catégorie est couplée à un anti-ligand spécifique d'un site ligand E2 de l'analyte, et l'on utilise aussi dans la réaction un anti-ligand spécifique d'un site E3 dudit analyte, cet anti-ligand étant couplé
- soit au même composé fluorophore F2 (traceur direct),
- soit au site ligand E1 utilisé sur les microsphères de calibration (traceur indirect), et l'on utilise en outre dans la réaction un traceur de révélation anti-ligand E1 couplé au composé fluorophore F2 (calibration hétérologue),
   iii) ladite seconde catégorie est couplée à un anti-ligand spécifique d'un site ligand E2 de l'analyte, et l'on utilise aussi dans la réaction un ligand qui est un analogue de l'analyte portant un site ligand E2, cet analogue étant couplé
- soit au même composé fluorophore F2 (traceur direct),
- soit au site ligand E1 utilisé sur les microsphères de calibration (traceur indirect), et l'on utilise en outre dans la réaction un traceur de révélation anti-ligand E1 couplé au composé fluorophore F2 (calibration hétérologue),
   iiii) ladite seconde catégorie est couplée à un analyte ou à un analogue de l'analyte portant un site ligand E2 et, l'on utilise aussi dans la réaction un anti-ligand E2, cet anti-ligand étant couplé
- soit au même composé fluorophore F2 (traceur direct),
- soit au site ligand E1 utilisé sur les microsphères de calibration (traceur indirect), et l'on utilise en outre dans la réaction un traceur de révélation anti-ligand E1 couplé au composé fluorophore F2 (calibration hétérologue).

Les modalités de mise en oeuvre ci-dessus ne sont pas nécessairement exclusives l'une de l'autre.

Dans des conditions préférentielles de mise en oeuvre du procédé ci-dessus décrit, l'on utilise un traceur de révélation indirect qui est un anti-ligand E1 couplé à un composé fluorophore F2.

Dans des conditions préférentielles de mise en oeuvre du procédé ci-dessus décrit, les microsphères de calibration se distinguent qualitativement des microsphères de dosage (spécifiques d'un analyte donné) par l'intensité d'une fluorescence F1. L'appréciation quantitative des interactions ligand/anti-ligand formées sur les différentes catégories de microsphères est réalisée par lecture de l'intensité d'une seconde fluorescence F2. La lecture de deux fluorescences seulement permet donc d'attribuer les signaux d'étalonnage et de dosages à chacune des catégories de microsphères mises en oeuvre dans le dosage.

Pour le dosage concomitant de 2 analytes, on utilise selon la présente invention, selon la modalité i) précédemment décrite, un deuxième ensemble formé par une deuxième catégorie de microsphères de calibration et une deuxième catégorie de microsphères de dosage ainsi que le ou les traceur(s) nécessaire(s). Selon les modalités ii), iii) et iiii), une deuxième catégorie de microsphères de calibration n'est pas nécessaire.

Chacune des catégories de microsphères de dosage ou de calibration utilisée pour le dosage du deuxième analyte doit comporter une caractéristique mesurable permettant de distinguer cette catégorie de microsphères des autres catégories de microsphères mises en oeuvre. On utilise de préférence la même caractéristique pour effectuer la discrimination ; par exemple si l'on a utilisé la fluorescence F1 pour distinguer les catégories de microsphères précédentes, on utilise aussi cette fluorescence F1, à une intensité différente, pour caractériser la nouvelle catégorie de microsphères. Par exemple, si la catégorie des microsphères de calibration est colorée à un niveau y de fluorescence F1 et la première catégorie de microsphères de dosage est colorée à un niveau 2y, la deuxième catégorie de microsphères de dosage sera à titre illustratif colorée à un niveau 3y.

On peut ainsi multiplier le nombre de couples de réactifs pour doser le nombre d'analytes différents souhaité.

Dans un mode préféré de mise en oeuvre de l'invention, on incube ensemble toutes les catégories de microsphères avec l'échantillon ; dans un autre mode préféré de mise en oeuvre de l'invention on mélange toutes les catégories de microsphères avec l'échantillon dès le début de la mise en oeuvre.

Lors de la mise en oeuvre du procédé, par exemple un mélange de microsphères comprenant la catégorie des microsphères de calibration ainsi que la ou les catégories de microsphères de dosages d'analytes que l'on souhaite mettre en oeuvre, est incubée dans l'échantillon inconnu en présence de l'anti-ligand du système de calibration et des anti-ligands correspondant aux catégories de microsphères précitées. Les réactions ligand/anti-ligands sont réalisées de façon simultanée ainsi que l'analyse subséquente des signaux.

De cette manière, chaque dosage d'échantillon génère d'une part, pour les microsphères de calibration, une série de signaux d'intensité discrète, en fonction de la quantité de ligands E1 présente à la surface des sous-catégories de microsphères de calibration, et d'autre part, sur chaque catégorie de microsphères réservée au(x) dosage(s), un signal unique qui est fonction de la quantité de substance inconnue que l'on souhaite déterminer.

Le résultat final établissant la concentration d'analyte dans l'échantillon est obtenu en exploitant les valeurs numériques des intensités de fluorescence F2 mesurées de façon simultanée sur les microsphères de dosage et de calibration.

La détection des interactions ligand/anti-ligands de calibration ou de mesure se fera soit directement par marquage fluorescent des anti-ligands correspondants, soit indirectement par un réactif fluorescent liant les anti-ligand ou les ligands liés à la surface des microsphères de calibration ou de mesure.

Dans des conditions préférentielles de mise en oeuvre du procédé ci-dessus décrit, la première catégorie de microsphères comprend au moins deux sous-catégories de microsphères, ces deux sous-catégories se distinguant l'une de l'autre par leur quantité de sites ligands E1.

La présente invention a notamment pour objet un procédé ci-dessus dans lequel l'on met en oeuvre l'une des modalités suivantes : i), ii), iii); i), ii) iiii); ii),iii),iiii); i),iii),iiii); i),ii); ii),iii); iii), iiii);i), iii); i); iii; iiii); ii),iiii); ii. Certaines des modalités i), ii), iii) et iiii) procédé ci-dessus peuvent être combinées dans la même mise en oeuvre du procédé, comme on l'a déjà indiqué.

Selon l'invention, l'étalonnage est de préférence réalisé simultanément à la détermination même du signal de mesure d'un analyte en utilisant un même système de détection et les fluorescences sont analysées sur chaque microsphère successivement, par le système de détection, en sorte que les mesures effectuées sur l'ensemble des microsphères de calibration et sur l'ensemble des microsphères de dosage mises en jeu lors du dosage d'un échantillon, soient statistiquement équivalentes ou qu'en d'autres termes les fluctuations du détecteur soient compensées par la survenue aléatoire des microsphères devant le détecteur, quelle que soit leur catégorie, dans un intervalle de temps donné.

Par « interaction spécifique ligand/anti-ligand », l'on entend une interaction de l'analyte, ou du composé servant à la calibration (ligand) avec une molécule partenaire (anti-ligand) qui ne réagit avec aucun autre constituant du milieu réactionnel (d'incubation).

Par « procédé de dosage », on entend de manière non limitative, les immunodosages d'analytes en sandwich, mettant en jeu chacun une paire d'anticorps spécifiques de l'analyte dont l'un est marqué (dosage de la thyréostimuline par exemple), les immunodosages par compétition, mettant en jeu soit un anticorps anti-analyte lié à la surface des microsphères et l'analyte marqué en traceur, soit l'analyte lié à la surface des microsphères et l'anticorps anti-analyte marqué en traceur (dosage de la thyroxine ou de l'oestradiol par exemple). D'autres systèmes de dosages mettant en jeu les interactions d'acides nucléiques synthétiques et d'acides nucléiques naturels ayant subi ou non une étape d'amplification enzymatique peuvent être avantageusement étalonnés selon la présente invention.

Par « analyte », l'on entend par exemple un sucre, un polypeptide, une protéine, et plus particulièrement une hormone telle que celle appartenant aux groupes des hormones thyroïdiennes (thyroxine, triiodothyronine), des hormones stéroïdes (cortisol, oestradiol, testostérone, ...etc.), des neurohormones (histamine, sérotonine, catécholamines, métanéphrines...etc). L'analyte peut être un polypeptide tel que par exemple une hormone hypophysaire comme l'hormone thyréostimuline (TSH), ou encore l'hormone de croissance (hGH), la corticotropine (ACTH), la parathormone (PTH), la somatotropine (hGH). L'analyte peut être plus particulièrement une cytokine telle que celles appartenant aux diverses familles de molécules endogènes comme les chemokines, lymphokines, monokines, interleukines, interférons, facteurs de colonisation, facteurs de croissance, ou neuro-peptides, et plus particulièrement l'interféron-α (IFN-α), l'interféron-β (IFN-β), l'interféron-γ, (IFN-γ), l'interleukine-1 (IL-1), l'interleukine-2 (IL-2), l'interleukine-3 (IL-3), l'interleukine-4 (IL-4), l'interleukine-5 (IL-5), l'interleukine-6 (IL-6), l'interleukine-10 (IL-10), le tumor necrosis factor (TNF), le transforming growth factor-β (TGF-β), les colony-stimulating factors (CSF) tels que le granulocyte macrophage-stimulating factor (GM-CFS) et le macrophage stimulating factor (M-CSF), le facteur de croissance épithélial (EGF), la somatostatine, les endorphines, les divers "releasing factors" ou "inhibitory factors" tels que le TRF. L'analyte peut également être un médiateur intra-cellulaire comme par exemple l'adénosine 3', 5'-monophosphate cyclique (CAMP) ou la guanosine 3', 5'-monophosphate cyclique (cGMP).

L'analyte peut être aussi une protéine constituante d'une particule virale comme par exemple la protéine p24 du virus HIV ou la protéine de surface du virus de l'hépatite B. L'analyte peut être aussi un médicament ou un de ses métabolites. Ce médicament peut être par exemple la théophylline, la digoxine, le méthotrexate ou la cyclosporine. L'analyte peut être représenté par une population d'anticorps présents dans l'échantillon comme par exemple les anticorps anti-organismes infectieux, les anticorps anti-constituants des particules virales HIV1, HIV2, ou des anticorps de type IgE ou lgG4 anti-allergène comme les anticorps anti-acariens ou les anticorps anti-pollens ou encore les anticorps anti-constituants du soi comme les anticorps anti-insuline, anti-récepteur de la TSH, anti-thyroperoxidase, anti-constituants nucléaires (anti-RO/SSA, anti-RNP, ...etc) ou anti-tissus (anti-mitochondries, anti-estomac, ...etc).

L'analyte peut-être aussi une séquence de DNA ou de RNA représentant le génome lui-même ou un transcript de ce même génome d'un virus comme par exemple le virus HIV ou HCV ou représentant celui d'une bacterie comme par exemple Mycobacterium tuberculosis ou Chlamydiae trachomatis ou représentant un oncogène résultant d'une modification du génome humain comme par exemple l'oncogène BCR/abl présent lors de la leucémie myélomateuse chronique.

Par "site ligand " l'on entend dans le cadre de la présente invention non seulement un épitope tel un fragment peptidique, mais encore plus généralement un site capable d'interagir spécifiquement avec un composé particulier pour former un couple ligand/anti-ligand. Le modèle le plus connu de couple ligand/anti-ligand est le couple antigène/anticorps mais on peut citer aussi les interactions acides nucléiques/acides nucléiques complémentaires, les interactions enzyme/inhibiteur,...etc. On peut citer par exemple à titre de site ligand de type antigène un site hapténique ou encore un peptide comportant de 5 à 50 acides aminés ou un sucre. Dans les acides nucléiques, on peut citer aussi un fragment de molécule d'ADN ou d'ARN comportant de 5 à 50 nucléosides.

Par « analogue d'analyte », l'on entend un composé de préférence structurellement proche qui peut entrer en compétition avec l'analyte original pour un anti ligand E2, voire E3.

Les échantillons sont par exemple un échantillon sanguin du type sérum, plasma, un échantillon d'urine ou de liquide céphalo-rachidien, un extrait cellulaire ou un surnageant de culture.

Par "composé fluorophore", l'on entend un composé détectable à une longueur d'onde donnée, par exemple la fluorescéine détectable à 520 nm ou le conjugué phycoérythrine-cyanine 5 (phycoérythrine-Cy5) détectable à 675 nm ; F1 ou F2 représente ci-après, selon le contexte, le composé fluorophore ou sa fluorescence, voire sa longueur d'onde.

Par « microsphères », l'on entend des supports particulaires solides formés à partir de polymères de matière plastique ou d'autres matières permettant l'immobilisation de molécules. Elles ne possèdent pas nécessairement le caractère sphérique qui est simplement le résultat de la plupart des procédés de fabrication. En particulier, on pourra utiliser des microsphères formées à partir de polymères de polysaccharides, de cellulose, de silicone, de Sephadex®, de silice, de gélatine, ou de paraffine. Les microsphères formées à partir de polymères de matières plastiques seront utilisées avantageusement. Ces dernières sont alors de diamètre déterminé allant de préférence de 0,5 à 40 µm et notamment de 3 à 10 µm environ. Ces particules sont réalisées par exemple par polymérisation contrôlée de monomères, de polystyrène, méthyl-métacrylate ou divinylbenzène pur ou en mélange. On peut citer par exemple les microsphères homogènes en tailles (5,5 µm) et présentant des fonctions amines libres (-NH2) en surface commercialisées par Dyno Particles, (Lilleström, Norvège). Les microsphères sont colorées, lors de leur fabrication, par incorporation du colorant dans le mélange de monomères ou, après la fabrication des microsphères, par imprégnation ou encore par couplage covalent en surface. On peut utiliser des particules colorées par exemple par la fluorescéine, par les molécules de la famille BODIPY®, par l'Oregon Green®, par la rhodamine, par la tétra-méthylrhodamine, par l'acridine orange, par les phycobilliprotéines (r ou b-phycoérythrine, allophycocyanine) conjuguées ou non à une cyanine (phycoérythrine-Cy5), ainsi que par les molécules de la série des cyanines, modifiées ou non. L'on entend aussi des supports particulaires solides tels que des cellules ayant subi un traitement de fixation ou des liposomes par exemple.

Les propriétés des microsphères selon l'invention sont d'être fonctionnalisables pour les rendre réactives avec le milieu biologique (par la fixation stable du ligand ou de l'anti-ligand), d'être détectables directement ou après traitement par un colorant et d'être de préférence suffisamment homogènes pour permettre une discrimination nette entre les différentes catégories à détecter.

Les microsphères de calibration se distinguent des microsphères de dosage sur le plan de la taille, du spectre d'émission, ou de l'intensité de la fluorescence F1. C'est ainsi par exemple que si, à taille égale, la catégorie des microsphères de calibration a une fluorescence particulière, d'une certaine intensité, détectable à une longueur d'onde donnée, on pourra par exemple utiliser la catégorie des microsphères de dosage colorées, de façon significativement plus intense à cette même longueur d'onde. Il est à noter que les autres paramètres de toutes les microsphères appartenant à une même catégorie doivent être identiques, notamment par leur taille, leur fluorescence et l'intensité de leur fluorescence.

La catégorie des microsphères de calibration se décompose en au moins une, notamment au moins 2, de préférence 3, plus particulièrement 4 sous-catégories. Ces sous-catégories se distinguent les unes des autres uniquement par les quantités de sites ligands E1 qu'elles portent. C'est ainsi par exemple que si l'on utilise 4 sous-catégories, à titre illustratif la première sous-catégorie ne comprend pas de site ligand E1, la seconde comprend x sites ligands E1, la troisième comprend 2x sites ligands E1, et la dernière sous-catégorie 4x sites ligands E1. Ces sites ligands doivent bien évidemment être accessibles à leurs anti-ligands spécifiques et c'est pourquoi ils sont situés en surface des microsphères de sorte que les traceurs formés par un anti-ligand spécifique du site ligand E₁ couplé à un composé fluorophore F2 vont se fixer sur les microsphères de calibration.

Le paramètre de discrimination des sous-catégories ci-dessus, c'est à dire dans l'exemple précité, l'intensité de fluorescence F2, est avantageusement choisi pour que, dans les conditions du dosage, les sous-catégories procurent des signaux distincts et couvrant de préférence toute la plage de valeurs possibles du signal qui peuvent être produites par la catégorie des microsphères de dosage lors de la mise en oeuvre de la méthode sur un échantillon inconnu.

Les microsphères de dosages sont caractérisables par la taille, la fluorescence ou l'intensité de la fluorescence, c'est-à-dire que toutes les microsphères de cette catégorie correspondant à un analyte donné partagent une même caractéristique, par exemple la même taille ou la même fluorescence F1, de manière à pouvoir être détectées par un dispositif de mesure approprié et distinguées sans ambiguïté des microsphères de calibration et des microsphères de dosages pour les autres analytes.

Dans une réalisation particulière de l'invention les sites ligands E1 et les anti-ligands E1 n'existent pas naturellement dans les fluides biologiques et notamment dans les échantillons d'origine humaine. On utilise par exemple un des couples DTPA Indium (diéthylène triamine penta acétique acide)/anti-DTPA Indium, succinyl-glycyl-histamine (SG-histamine)/anti-SG-histamine, 2-4 dinitrophénol (DNP)/anti-DNP et d'une manière générale tout motif synthétique de masse moléculaire comprise entre 300 et 1000 et l'anti-ligand qui le fixe spécifiquement.

Lors de la calibration de systèmes multianalytiques regroupant des réactifs de dosages constitués par des couples ligand/anti-ligand caractérisés par des enthalpies de réactions comparables, pour des dosages à l'équilibre, ou par des propriétés cinétiques similaires, s'il s'agit de systèmes hors équilibre, on choisira avantageusement un couple ligand E1/anti-ligand E1, caractérisé par une enthalpie de réaction ou par une cinétique de liaison la plus proche de celle des réactifs mis en jeu lors du dosage.

Par « traceur », l'on entend un conjugué formé par un ligand ou un anti-ligand et un fluorophore. Ces traceurs permettent de quantifier les interactions ligand/anti-ligands. Le marquage sera réalisé de préférence avec un fluorophore F2 unique et commun à toutes les interactions ligand/anti-ligands mises en oeuvre simultanément Si l'on a utilisé, par exemple, l'intensité de la coloration produite par un fluorophore F1 comme caractéristique pour distinguer entre elles les différentes catégories et sous catégories des microsphères de calibration et de dosage, on choisira un fluorophore d'une fluorescence F2 suffisamment différente de F1, c'est-à-dire de celle déjà utilisée pour réaliser le marquage des microsphères, pour que F1 et F2 soient séparables dans les voies de mesure de l'instrument de mesure utilisé. Par exemple, si l'on a utilisé l'intensité de la fluorescéine (F1, fluorescence verte) pour caractériser les différentes catégories et sous catégories de microsphères, on utilisera par exemple la phycoérythrine-Cy5 (F2, fluorescence rouge) pour caractériser les traceurs. Ce marquage sera réalisé de préférence par couplage covalent comme par exemple par activation de groupements chimiques présents sur le fluorophore ou sur le réactif à marquer.

Dans une première approche de l'invention (utilisation de traceurs directs) on détecte directement les interactions mises en jeu, lors de l'incubation simultanée des réactifs et de l'échantillon, en marquant directement par le fluorophore F2 les traceurs engagés dans les interactions spécifiques sur les microsphères de dosages et sur les microsphères de calibration respectivement.

Une deuxième approche consiste en la détection indirecte des interactions spécifiques à l'aide d'un système de marquage secondaire. Le principe est de marquer une des espèces engagées dans l'interaction spécifique ligand-anti-ligand par un motif qui n'est pas directement détectable (traceur indirect) mais qui est reconnu par un système ligand/anti-ligand secondaire, qui sera lui, marqué avec le fluorophore F2 (traceur de révélation). Un exemple de détection secondaire est un système haptène/anti-haptène tel que précédemment décrit. Un autre exemple de système de détection secondaire est le système biotine/avidine-phycoérythrine. Dans cette deuxième approche de la présente invention, pour les schémas par compétition, on pourra utiliser un analyte purifié ou un analogue de l'analyte purifié portant l'épitope E2 et marqué secondairement par la biotine par exemple, ou encore l'anti-ligand E2 marqué secondairement à la biotine si l'épitope E2 est lui-même lié à la catégorie de microsphères mises en jeu lors du dosage. Dans le cas d'un schéma de type sandwich, on marquera l'anti-ligand E3 avec le système de marquage secondaire biotine. De même, pour ce qui est des interactions mises en jeu sur les microsphères de calibration, le ligand E1 sera représenté par la biotine, dans l'exemple donné ci-dessus. De cette manière, un seul type de marqueur sera utilisé pour mettre en évidence toutes les interactions ligand/anti-ligand mises en jeu lors des dosages.

Dans d'autres conditions préférentielles de mise en oeuvre du procédé ci-dessus décrit, les microsphères de calibration et les microsphères de dosage sont de même taille.

Dans encore d'autres conditions préférentielles de mise en oeuvre du procédé ci-dessus décrit, le paramètre de discrimination des sous-catégories de microsphères de calibration entre elles est une intensité de fluorescence.

Dans toujours d'autres conditions préférentielles de mise en oeuvre du procédé ci-dessus décrit, les paramètres de discrimination des microsphères de calibration et de mesure pour chaque analyte sont des combinaisons d'intensité de fluorescence F1 et de taille.

A la fin de l'incubation de l'ensemble des réactifs (microsphères et traceurs) avec l'échantillon biologique, lorsque les réactions ligand/anti-ligand se sont réalisées, on procède alors à la mesure des paramètres caractéristiques, par exemple aux longueurs d'onde F1 et F2. Ces mesures sont faites à l'aide des appareils et techniques de détermination de la nature et de la quantité de particules habituellement utilisées dans l'état de la technique, c'est-à-dire principalement les cytomètres de flux. On peut, par exemple, citer les techniques et instruments décrits notamment dans les brevets US-A-3 925 018 et US-A-1 561 042. On peut également citer les cytomètres statiques à balayage LASER tels que celui proposé par la Société COMPUCYTE (Cambridge MA USA) et décrit dans les brevets US-A-5 072 382 ; 5 107 422 ;5 202 230; 5 427 910 et 5 523 207. De tels appareils sont capable s d'analyser individuellement et successivement plus de 10³ particules par seconde. Ces instruments doivent être munis d'au moins deux détecteurs aux longueurs d'onde d'émission des composés F1 et F2 et éventuellement d'un détecteur de lumière diffractée permettant de détecter la taille des particules.

La réalisation de l'invention s'opère en deux étapes:
- La première étape, ou «étape d'étalonnage», consiste en l'établissement du lien entre chaque sous-catégorie de microsphère de calibration Cal1, ...,Calp et une concentration d'analyte. Pour cela l'ensemble des réactifs, microsphères de calibration Cal1,...,Calp et microsphères de dosages (Dos), est incubé avec successivement des concentrations étalon CO1, ...,COn d'analyte et les traceurs correspondants. Si le réglage de l'appareil est stable et les conditions d'incubations strictement identiques on peut tracer la fonction d'interpolation qui lie le signal de mesure F2 aux concentrations d'analyte. On utilise ensuite cette fonction pour associer à chaque signal issu des microsphères de calibration une concentration d'analyte C1, ... ,Cp. Le tableau suivant résume l'ensemble des opérations:

| Concentration des étalons | Signaux de référence | | Signaux des microsphères de calibrations | Concentrations interpolées |
|---|---|---|---|---|
| CO1 | F2 (CO1) | Construction de la fonction d'interpolation | F2 (Cal1) | C1 |
| CO2 | F2 (CO2) | | F2 (CaI2) | C2 |
| · | · | | · | · |
| · | · | | · | · |
| · | · | | · | · |
| COn | F2 (COn) | | F2 (Calp) | Cp |

Le résultat de l'opération est l'association des concentrations C1, ...Cp aux calibrateurs Cal1, ...Calp. On note que le nombre de calibrateurs p n'a pas raison d'être identique au nombre d'étalons n. Si le réglage de l'instrument n'est pas suffisamment stable, on peut rechercher par la méthode des essais et des erreurs la concentration d'analyte C1 qui donne sur les microsphères de dosage le même signal que les microsphères de calibration Cal1 avec le traceur qui lui est associé, puis de même pour C2 et Cal2, etc...
On remarque aussi que l'opération peut être répétée pour plusieurs analytes, ainsi à une même série de calibrateurs pourra correspondre autant de tables de valeurs de concentration qu'il y a d'analytes dont on souhaite la détermination, ce qui ouvre la possibilité d'une calibration commune dans un système multianalytique.
- La deuxième étape, ou «étape de calibration», est celle qui s'opère chez l'utilisateur terminal. Le même mélange de microsphères Cal1,...Calp et Dos et les traceurs correspondants sont incubés avec l'échantillon inconnu, on enregistre les signaux F'2(Cal1)...F'2(Calp), qui n'ont aucune raison d'être les mêmes que ceux déterminés dans la phase 1, s'agissant d'un appareil différent et de conditions d'incubation qui peuvent aussi différer. Les signaux recueillis restent néanmoins associés aux concentrations d'analytes et peuvent être exploités pour tracer une fonction d'interpolation qui sert ensuite à calculer la concentration de l'échantillon inconnu:

| Microsphères de calibrations | Concentration associée | Signaux de calibration | | Signaux de l'inconnu | Concentration interpolée |
|---|---|---|---|---|---|
| Cal1 | C1 | F'2(Cal1) | Fonction d'interpolation | | |
| · | | · | | | |
| · | | · | | F2 | C |
| · | | · | | | |
| Calp | Cp | F'2(Calp) | | | |

Le système peut fonctionner en mode multianalytique si l'on dispose de plusieurs catégories de microsphères de dosage Dos1, Dos2, Dos3, correspondant à des analytes différentes. Les signaux des calibrateurs sont traduits en autant de fonctions d'interpolation qu'il y a d'analytes, au moyen des tables de concentration qui leur sont associées. Il est à noter que les schémas réactionnels peuvent être complètement différents d'un analyte à l'autre, les fonctions d'interpolation pouvant être croissantes ou décroissantes selon qu'il s'agit d'un schéma « sandwich » ou d'un schéma «compétition».

Dans une réalisation particulière de l'invention, on utilisera la comparaison des signaux obtenus sur les microsphères de calibration lors de l'étalonnage dans le laboratoire du fabricant (étape 1), et lors de la mise en oeuvre du dosage chez l'utilisateur (étape 2) de façon à corriger les signaux obtenus sur les microsphères de dosages dans cette dernière étape. Dans ce cas, la détermination de la concentration en analyte inconnu s'effectuera grâce la fonction d'interpolation construite lors de la première étape chez le fabricant. Un exemple de ce mode de calcul, donné dans le cas d'un calibration correspondant à l'art antérieur, est décrit en détail par M.J. Lynch dans «Journal of Bioluminescence and Chimiluminescence, vol4, pp615-619, 1989».

Une réalisation particulière de l'invention concerne les dosages de type «sandwich» à calibration homologue évoqués en i) ci-dessus, dans lesquels le système de dosage exploite au moins deux sites présents sur l'analyte, comme par exemple un dosage mettant en jeu deux anticorps monoclonaux liant deux épitopes distincts de l'analyte à doser ou un antigène déterminé et un anticorps anti-immunoglobuline, pour le dosage d'immunoglobulines spécifiques. Le système de calibration sera alors avantageusement constitué par une population de microsphères de calibration différentiable par le paramètre F1 et comportant à sa surface une quantité fixe de l'analyte considéré. Dans le cas où le système de dosage est formé par deux anti-ligands, l'un lié à la surface des microsphères de dosages, l'autre sous forme de traceur, il est important que sur les microsphères de calibration, l'analyte soit modifié (notamment par masquage ou délétion) de façon à rendre inaccessible le site reconnu par les microsphères de dosages. Dans cette réalisation particulière, le traceur permet à la fois la mesure et la calibration, car lors de l'incubation simultanée de ces deux catégories de microsphères avec l'échantillon à doser et avec l'anticorps traceur, ce dernier vient se lier à la surface des microsphères de calibration et de mesure.

La fixation de l'anticorps traceur sur les microsphères de mesure est fonction de la concentration d'antigène présente dans l'échantillon à doser alors que la fixation de l'anticorps traceur sur les microsphères de calibration est fixe et proportionnelle à la quantité de sites d'analyte que l'on aura préalablement liés en surface.

Dans une autre réalisation de l'invention, où la première catégorie de microsphères de calibration est constituée d'une seule sous-catégorie, la quantité de traceur se liant aux microsphères de calibration produit un signal de fluorescence exactement placé au point de coupure qui détermine si l'échantillon dosé est positif ou négatif pour l'analyte mesuré, par exemple pour la présence ou l'absence d'anticorps anti-pathogène (micro-organisme infectieux), anti-soi (tissus, hormone, protéines nucléaires), anti-allergène (pollens) ou d'antigène viral tel que l'antigène Hbs ou encore de marqueurs de cancers tels que des molécules solubles de la famille des antigènes carbohydrates. Ainsi, l'intensité du signal porté par les microsphères de mesure peut être qualifiée de positive ou de négative en toute exactitude. De même que la fixation non spécifique d'immunoglobuline irrelevante, qui ne manque pas d'interférer dans les dosages de sérologie, peut être quantifiée exactement sur les microsphères de calibration.

La présente demande a encore pour objet les réactifs et les kits de dosages qui permettent la réalisation de l'invention, c'est à dire la mesure de concentrations d'analytes dans un tube avec un étalonnage interne. Les réactifs et kits correspondent aux différents modes de réalisation de l'invention.

La présente demande a donc encore pour objet un kit de dosage mettant en jeu le procédé d'étalonnage tel que défini ci-dessus, comportant
- des microsphères de calibration
- des microsphères de dosage
- un traceur de calibration
- un traceur de dosage (servant aussi de traceur de calibration pour la modalité i), pour les modalités ii), iii) et iiii) tels que définis dans la revendication 1 et
- une table de correspondance microsphères de calibration - données numériques nécessaires à l'interprétation d'un signal mesuré.

Ces données numériques peuvent être la concentration d'analyte.

Ces données peuvent aussi correspondre au niveau de fluorescence d'étalonnage ainsi qu'aux paramètres de l'équation d'interpolation de référence obtenue lors de l'étalonnage de la méthode pour chaque analyte.

Elle a notamment pour objet un kit de dosage tel que défini ci-dessus, caractérisé en ce qu'il comprend les réactifs correspondant à l'une des modalités i), ii), iii);et iiii) précédemment définies.

Dans des conditions préférentielles de mise en oeuvre des kits ci-dessus décrits, on utilise les conditions indiquées ci-dessus comme avantageuses pour le procédé.

La présente demande a enfin pour objet un procédé de mesure ou de détection d'un seuil d'un analyte mettant en jeu un procédé d'étalonnage ou un kit ci-dessus.

### PARTIE EXPERIMENTALE

### Exemple 1 - Préparation des réactifs

### A- Obtention des microsphères de différentes catégories.

Différentes catégories de microsphères sont obtenues à partir de microsphères homogènes en taille (5,5 µm) et présentant des fonctions amines libres (-NH2) en surface (Dyno particles, Lilleström, Norvège). Elles sont lavées et mises en suspension dans un tampon phosphate 0,5 moles/l, pH 6,5. Trois aliquotes identiques de ces microsphères sont préparées. Chaque aliquote est incubée avec un mélange défini de biotine-n-hydroxysuccinimide (biotine-NHS) et de fluorescéine-N-hydroxysuccinimide (fluorescéine-NHS) pendant 2 heures à température ambiante et sous agitation. Les 3 mélanges différents comprennent respectivement 0 moles, 7,87 x 10⁻¹⁰ moles et 3,15 x 10⁻⁹ moles de fluorescéine-NHS pour 10 mg de microsphères, la quantité de biotine-NHS restant constante (0,575 x 10⁻⁶ moles).

Les microsphères biotinylées et fluorescentes sont ensuite lavées plusieurs fois dans le même tampon et conservées à 4°C à l'abri de la lumière. Les trois catégories sont dénommées respectivement, microsphères de calibration (pas de fluorescéine en surface), microsphères de dosage N° 1 incubées avec 7,87.10⁻¹⁰ moles de fluorescéine-NHS et microsphères de dosage N° 2 incubées avec 3,15.10⁻⁹ moles de fluorescéine-NHS.

### Obtention de microsphères avidinées.

Les microsphères de calibration et les microsphères de dosage N° 1 et 2 sont incubées séparément avec une solution de streptavidine à 250 µg pour 10 mg de microsphères en tampon phosphate 8 mmoles/l, azoture de sodium 10 mmoles/l, pH 7,2 pendant 18 heures à 4°C et sous agitation. Après 3 lavages dans un tampon phosphate 8 mmoles/l, 1g/l d'albumine bovine (BSA), 10 mmoles/l azoture de sodium pH 7,2 (tampon phosphate-BSA), la solution de microsphères avidinées est conservée, pour une utilisation ultérieure, dans ce même tampon.

### Obtention du conjugué 2,4-dinitrophenyl-BSA-biotine.

Le conjugué 2,4-dinitrophenyl-BSA-biotine, (DNP-BSA-biotine) est obtenu en faisant réagir d'abord de la biotine-n-hydroxysuccinimide sur de la BSA diluée en tampon borate 20 mmoles/l, NaCI 50 mmoles/l, pH 8,5 (tampon borate) avec un rapport 1 mole de BSA pour 5,6 moles de biotine-NHS puis après avoir séparé la biotine n'ayant pas réagi, en incubant, en tampon borate, de l'acide 6-(2,4-dinitrophenyl)aminohexanoïque succinimidyl ester (Molecular Probes Europe, Hollande) (DNP-NHS) à un rapport de 1 mole de BSA biotinylée pour 10 moles de DNP-NHS. Le DNP-NHS n'ayant pas réagi est éliminé.

### Obtention de microsphères de calibration.

La solution de microsphères de calibration avidinées, est divisée en 4 aliquotes. Chaque aliquote est incubée pendant 18 heures à 4°C sous agitation avec une quantité donnée de conjugué DNP-BSA-biotine dilué en tampon phosphate-BSA. La première sous-catégorie de microsphères (Cal.1) est incubée sans DNP-BSA-biotine. La deuxième sous-catégorie (Cal.2) est obtenue en incubant 0,062 µg/ml de conjugué DNP-BSA-biotine, la troisième, (Cal.3) 0,25 µg/ml et la quatrième (Cal.4.), 0,6 µg/ml. A l'issue de l'incubation, les microsphères sont lavées plusieurs fois en tampon phosphate 50 mmoles/l pH 7,2 puis une solution de diméthylsubérate (0,2 µmoles par mg de microsphères) est ajoutée à chaque catégorie de microsphères de calibration. Après une heure de réaction à température ambiante, les 4 solutions de microsphères de calibration sont lavées en tampon phosphate-BSA puis diluées dans ce même tampon à 4,10⁶ microsphères par ml et mélangées à parties égales.

### Obtention du traceur de calibration.

Un anticorps monoclonal anti-DNP (clone n°134, Immunotech, France) est conjugué à la phycoérythrine en utilisant un réactif hétéro-bifonctionnel de type sulfo-SMCC (Pierce Chemical, Oud-Bejerland, Hollande) selon les instructions données par le fabricant du réactif. Le produit de la réaction est purifié par chromatographie d'exclusion sur une colonne de type R 10/60 (Pharmacia, Uppsala, Suède). Les fractions, montrant la double activité de reconnaissance du motif DNP et d'émission de fluorescence, caractéristique de la phycoérythrine, sont sélectionnées et mélangées ensemble. On évitera de sélectionner les fractions éluées les premières de manière à ne pas utiliser les polymères de haut poids moléculaires dans la solution de traceur.

### Obtention des microsphères et des traceurs de dosage.

Pour chaque analyte à doser (par exemple l'IL4 ou l'IFNγ) on utilise un couple d'anticorps, reconnaissant chacun un épitope distinct du même analyte.

Un premier anticorps anti-lL4 est sélectionné pour être immobilisé sur les microsphères de dosage N°1. L'anticorps est alors biotinylé en faisant réagir de la biotine-NHS, en tampon borate, avec un rapport de 1 mole d'anticorps pour 13 moles de biotine-NHS. La biotine n'ayant pas réagi est séparée par chromatographie sur Sephadex® G25 (Pharmacia, Uppsala Suède). L'anticorps biotinylé anti-lL4 est alors immobilisé sur les microsphères de dosage 1 en incubant, en phosphate-BSA, l'anticorps à 1µg par mg de microsphères. Après 18 heures d'incubation sous agitation, les microsphères sont lavées et diluées à 10⁶ microsphères par ml dans ce même tampon.

Le second anticorps anti-lL4 est couplé à la phycoérythrine selon le protocole décrit plus haut pour l'anticorps anti-DNP.

On procède de même pour les anticorps anti- l'IFNγ.

On effectue les opérations ci-dessus pour l'IL4 et l'IFNγ. On obtient ainsi 4 réactifs immunologiques
- un anticorps spécifique de l'IL4, dirigé contre un premier épitope de cette molécule et couplé à des microsphères marquées à la fluorescéine,
- un anticorps spécifique de l'IL4, dirigé contre un second épitope de cette molécule et couplé à la phycoérythrine,
- un anticorps spécifique de l'IFNγ, dirigé contre un premier épitope de cette molécule et couplé à des microsphères marquées à la fluorescéine,
- un anticorps spécifique de l'IFNγ, dirigé contre un second épitope de cette molécule et couplé à la phycoérythrine.

### Exemple 2 : Dosage de l'IL4 et de l'IFNγ dans un échantillon

### A. Procédure

Les microsphères de calibration et les microsphères de dosage (N° 1 et N° 2) sont mélangées de manière à obtenir une concentration de microsphères d'environ 15 000 microsphères par catégorie dans 10 µl de tampon phosphate-BSA. Les traceurs anti-DNP, anti-lL4, anti-IFNγ sont mélangés de manière à ce que chaque traceur soit à la concentration de 25 ng dans 50 µl de tampon phosphate-BSA. Les concentrations respectives de traceurs et de microsphères peuvent être ajustées en fonction des caractéristiques de dosage souhaitées.

Cinquante microlitres de l'échantillon sont déposés dans un puits de microplaque à fond filtrant (NUNC, Roskilde, Denmark). Dix microlitres du mélange des microsphères sont ajoutés puis 50 µl du mélange des traceurs. Le milieu réactionnel est maintenu sous agitation forte pendant deux heures à température ambiante.

A l'issue de l'incubation, le milieu réactionnel est filtré et les microsphères sont reprises dans un liquide de lavage contenant 0,15 moles/l de NaCI et 0,05% de Tween 20. Cette étape est renouvelée deux fois.

Puis, les microsphères sont reprises dans du tampon phosphate 50 mmoles/l, pH 7,8 et sont injectées sur un cytomètre de flux (Epics XL, Coulter Corp., Miami, USA). L'acquisition est stoppée lorsqu'environ 500 microsphères par catégorie ont été enregistrées. Les photomultiplicateurs de discrimination (520 nm) et de détection (675 nm) sont réglés de façon à discriminer les trois populations de microsphères de calibration et les microsphères de dosage 1 et 2 selon leurs intensités de fluorescence respectives à 520 nm (émission de la fluorescéine) et de façon à avoir le plus grand rapport signal de mesure sur bruit de fond à 675 nm (émission de la phycoérythrine-Cy5).

### B. Résultats

Dans une série d'expérimentations, dans laquelle on a dosé des solutions étalons d'lL4 et d'IFNγ selon le protocole décrit ci-dessus, on a déterminé les correspondances entre les valeurs d'intensités des sous-catégories de microsphères de calibration et les concentrations en cytokines IL4 et IFNγ. Les résultats sont montrés dans le tableau 1 ci-dessous.

**Tableau 1**

| Microsphères de calibration | Concentration associée en IL4 (pg/ml) | Concentration associée en IFNγ (pg/ml) |
|---|---|---|
| Cal. 1 | 0,00 | 0,00 |
| Cal. 2 | 31,47 | 42,00 |
| Cal. 3 | 183,00 | 248,00 |
| Cal. 4 | 1110,01 | 1758,00 |

Ce tableau de correspondance permet de réaliser l'étalonnage de chaque système de dosage, IL4 et IFNγ. Les valeurs correspondantes en pg/ml sont utilisées pour déterminer la concentration des cytokines IL4 et IFNγ dans un échantillon inconnu.

### C. Exemple de dosage de l'IL4 et de l'IFNγ

Dans une autre série d'expérimentations, on a dosé, selon le protocole décrit précédemment, des échantillons constitués par des surnageants de culture de lymphocytes stimulés. Les concentrations en cytokines IL4 et IFNγ ont été déterminées avec, soit les microsphères de calibration, soit avec une gamme de solutions étalons. Les résultats sont donnés dans les tableaux 2 et 3 ci-dessous :

**Tableau 2**

| SURNAGEANT N° | Concentration en IL4 (Présente invention) (pg/ml) | Concentration en IL4 (Art antérieur) (pg/ml) |
|---|---|---|
| 1 | 5,68 | 7,68 |
| 2 | 31,72 | 31,77 |
| 3 | 61,72 | 67,16 |
| 4 | 109,51 | 121,93 |

**Tableau 3**

| SURNAGEANT N° | Concentration en IFNγ (Présente invention) (pg/ml) | Concentration en IFNγ (Art antérieur) (pg/ml) |
|---|---|---|
| 1 | 27,6 | 32,3 |
| 2 | 90,9 | 100,6 |
| 3 | 237,8 | 239,4 |
| 4 | 254,3 | 256,3 |

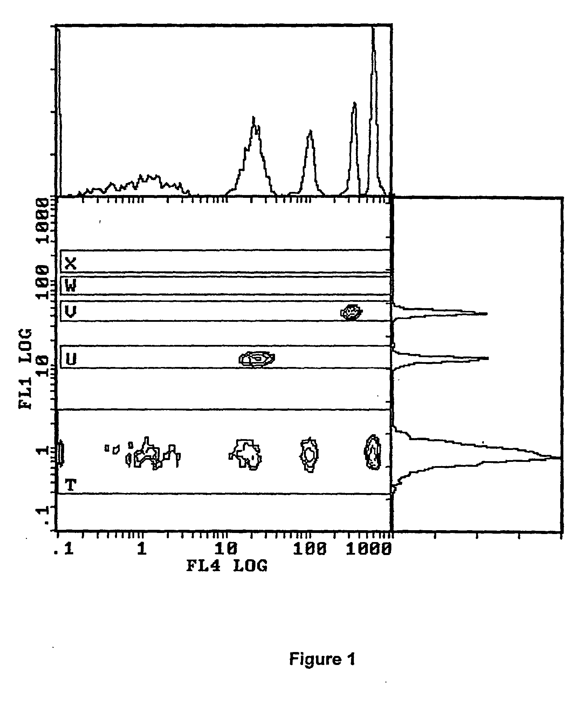

La Figure 1 est un histogramme d'acquisition typique obtenu à l'aide d'un cytomètre COULTER XL illustrant les résultats d'un dosage simultané de l'IL4 et de l'IFNγ, ainsi que le système d'étalonnage selon les conditions décrites dans l'exemple ci-dessus. Le canal T contient les 4 sous-catégories de microsphères de calibration engendrant 4 points. Elles sont réparties, en abscisse, sur l'ensemble de l'échelle de mesure. Les canaux U et V montrent respectivement la population de microsphères du système de dosage de l'IL4 et de l'IFNγ (microsphères de dosage N° 1 et 2). Leur position en abscisse est proportionnelle à la quantité d'lL4 et d'lFNγ. Les valeurs moyennes correspondant à l'ensemble des populations de microsphères individualisées sur le schéma sont obtenus par le logiciel d'analyse de l'instrument.

Légende : abscisse, FL4 fluorescence à 675 nm; ordonnée, FL1: fluorescence à 520 nm

## Revendications

1. Un procédé d'étalonnage d'un système de dosage d'un analyte dans un échantillon biologique, mettant en jeu une interaction spécifique de type ligand/anti-ligand dans un schéma réactionnel de type «sandwich» ou «compétition», **caractérisé en ce que** l'on fait réagir l'échantillon biologique avec un mélange de microsphères comprenant
- une première catégorie de microsphères caractérisable par sa taille, le spectre de fluorescence (F1) ou par l'intensité de la fluorescence (F1), appelées microsphères de calibration, ces microsphères comportant un site ligand E1, et avec
- un traceur formé par un anti-ligand spécifique du site ligand E₁ et couplé à un composé fluorophore F2 d'une fluorescence différente de celle (F1) qui peut caractériser les microsphères de calibration et avec
- une seconde catégorie de microsphères, appelées microsphères de dosages, différente de la première catégorie de microsphères sur le plan ou de la taille, ou du spectre de fluorescence (F1) ou de l'intensité de la fluorescence (F1), et qui constitue le support de la réaction analytique, selon l'une des modalités de mise en oeuvre suivantes:
i) dans le cas où l'analyte comprend un site E1 identique à celui porté par les microsphères de calibration, ladite seconde catégorie est couplée à un anti-ligand spécifique d'un site ligand E2 de l'analyte, et l'on utilise aussi dans la réaction un traceur anti-ligand E1 couplé au composé fluorophore F2 (calibration homologue),
ii) ladite seconde catégorie est couplée à un anti-ligand spécifique d'un site ligand E2 de l'analyte, et l'on utilise aussi dans la réaction un anti-ligand spécifique d'un site E3 dudit analyte, cet anti-ligand étant couplé
- soit au même composé fluorophore F2 (traceur direct),
- soit au site ligand E1 utilisé sur les microsphères de calibration (traceur indirect), et l'on utilise en outre dans la réaction un traceur de révélation anti-ligand E1 couplé au composé fluorophore F2 (calibration hétérologue),
iii) ladite seconde catégorie est couplée à un anti-ligand spécifique d'un site ligand E2 de l'analyte, et l'on utilise aussi dans la réaction un ligand qui est un analogue de l'analyte portant un site ligand E2, cet analogue étant couplé
- soit au même composé fluorophore F2 (traceur direct),
- soit au site ligand E1 utilisé sur les microsphères de calibration (traceur indirect), et l'on utilise en outre dans la réaction un traceur de révélation anti-ligand E1 couplé au composé fluorophore F2 (calibration hétérologue),
iiii) ladite seconde catégorie est couplée à un analyte ou à un analogue de l'analyte portant un site ligand E2 et, l'on utilise aussi dans la réaction un anti-ligand E2, cet anti-ligand étant couplé
- soit au même composé fluorophore F2 (traceur direct),
- soit au site ligand E1 utilisé sur les microsphères de calibration (traceur indirect), et l'on utilise en outre dans la réaction un traceur de révélation anti-ligand E1 couplé au composé fluorophore F2 (calibration hétérologue).

2. Un procédé d'étalonnage selon la revendication 1 **caractérisé en ce que** l'on utilise un traceur de révélation indirect qui est un anti-ligand E1 couplé à un composé fluorophore F2.

3. Un procédé d'étalonnage selon la revendication 1 ou 2 **caractérisé en ce que** la première catégorie de microsphères comprend au moins deux sous-catégories de microsphères, ces deux sous-catégories se distinguant l'une de l'autre par leur quantité de sites ligands E1.

4. Un procédé d'étalonnage selon la revendication 1 2 ou 3 **caractérisé en ce que** l'étalonnage est réalisé simultanément à la détermination même du signal de mesure d'un analyte en utilisant un même système de détection et **caractérisé en ce que** les fluorescences sont analysées sur chaque microsphère successivement, par un système de détection, en sorte que les mesures effectuées sur l'ensemble des microsphères de calibration et sur l'ensemble des microsphères de dosage mises en jeu lors du dosage d'un échantillon, soient statistiquement équivalentes.

5. Un procédé d'étalonnage selon l'une des revendications 1 à 4 **caractérisé en ce que** les différentes réactions de type ligand anti-ligand mise en jeu sont de nature antigène-anticorps.

6. Un procédé d'étalonnage selon l'une des revendications 1 à 4 **caractérisé en ce que** les différentes réactions de type ligand-anti-ligand mise en jeu sont de nature acide nucléique-acide nucléique complémentaire.

7. Un procédé d'étalonnage selon l'une des revendications 1 à 4 **caractérisé en ce que** les différentes réactions de type ligand-anti-ligand mises en jeu sont de nature enzyme-inhibiteur.

8. Un procédé d'étalonnage selon l'une des revendications 1 à 7 **caractérisé en ce que** les microsphères de calibration et les microsphères de dosage sont de même taille.

9. Un procédé d'étalonnage selon l'une des revendications 3 à 8 **caractérisé en ce que** le paramètre de discrimination des sous-catégories de microsphères de calibration entre elles est une intensité de fluorescence.

10. Un procédé d'étalonnage selon l'une des revendications 1 à 7 et 9 **caractérisé en ce que** les paramètres de discrimination des microsphères de calibration et de mesure pour chaque analyte sont des combinaisons d'intensité de fluorescence F1 et de taille.

11. Un kit de dosage mettant en jeu le procédé d'étalonnage tel que défini à l'une des revendications 1 à 10 comportant
- des microsphères de calibration
- des microsphères de dosage
- un traceur de calibration
- un traceur de dosage (servant aussi de traceur de calibration pour la modalité i), pour les modalités ii), iii) et iiii) tels que définis dans le revendication 1 et
- une table de correspondance microsphères de calibration - données numériques nécessaires à l'interprétation d'un signal mesuré.

12. Un kit de dosage selon la revendication 11, **caractérisé en ce qu'**il comprend les réactifs correspondant à l'une des modalités i), ii), iii);et iiii) telles que définies à la revendication 1.

## Patentansprüche

1. Verfahren zum Kalibrieren eines Systems für die Dosierung eines Analyten in einer biologischen Probe unter Anwendung einer spezifischen Wechselwirkung vom Ligand/Antiligand-Typ in einem Reaktionsschema vom "Sandwich"- oder "Konkurrenz"-Typ, **dadurch gekennzeichnet, dass** die biologische Probe mit einer Mikrokugelmischung reagieren gelassen wird, welche folgendes umfasst:
- eine erste, aufgrund von Größe, Spektrum der Fluoreszenz (F1) oder Intensität der Fluoreszenz (F1) charakterisierbare Kategorie von Mikrokugeln, genannt Kalibrier-Mikrokugeln, welche Mikrokugeln eine Ligandenstelle E1 aufweisen, und mit
- einem Tracer, der durch einen spezifischen Antiliganden der Ligandenstelle E1 gebildet und an eine Fluorophorverbindung F2 mit einer anderen Fluoreszenz als jene (F1) zur Charakterisierung der Kalibrier-Mikrokugeln gebunden ist, und mit
- einer zweiten Kategorie von Mikrokugeln, genannt Dosierungs-Mikrokugeln, die sich von der ersten Kategorie von Mikrokugeln entweder hinsichtlich Größe oder Spektrum der Fluoreszenz (F1) oder Intensität der Fluoreszenz (F1) unterscheidet und den Träger der Analysenreaktion bildet, u.zw. gemäß einer der folgenden Durchführungsbedingungen:
i) für den Fall, dass der Analyt eine Stelle E1 aufweist, die identisch mit jener auf den Kalibrier-Mikrokugeln ist, wird die zweite Kategorie an einen spezifischen Antiliganden einer Ligandenstelle E2 des Analyten gebunden, und es wird auch bei der Reaktion ein Antiligand-E1-Tracer verwendet, der an die Fluorophorverbindung F2 gebunden ist (homologe Kalibrierung),
ii) die zweite Kategorie wird an einen spezifischen Antiliganden einer Ligandenstelle E2 des Analyten gebunden, und es wird auch bei der Reaktion ein spezifischer Antiligand einer Stelle E3 dieses Analyten verwendet, wobei dieser Antiligand gebunden ist
- entweder an dieselbe Fluorophorverbindung F2 (direkter Tracer)
- oder an die Ligandenstelle E1, die an den Kalibrier-Mikrokugeln verwendet wird (indirekter Tracer), und außerdem wird bei der Reaktion ein an die Fluorophorverbindung F2 gebundener Antiligand-E1-Entwicklungstracer verwendet (heterologe Kalibrierung),
iii) die zweite Kategorie wird an einen spezifischen Antiliganden einer Ligandenstelle E2 des Analyten gebunden, und es wird auch bei der Reaktion ein Ligand verwendet, der ein Analog des eine Ligandenstelle E2 aufweisenden Analyten ist, wobei dieses Analog gebunden ist
- entweder an dieselbe Fluorophorverbindung F2 (direkter Tracer)
- oder an die Ligandenstelle E1, die an den Kalibrier-Mikrokugeln verwendet wird (indirekter Tracer), und außerdem wird bei der Reaktion ein Antiligand-E1-Entwicklungstracer verwendet, der an die Fluorophorverbindung F2 gebunden ist (heterologe Kalibrierung),
iiii) die zweite Kategorie wird an einen Analyten oder an ein Analog des eine Ligandenstelle E2 aufweisenden Analyten gebunden, und es wird auch in der Reaktion ein Antiligand E2 verwendet, wobei dieser Antiligand gebunden ist
- entweder an dieselbe Fluorophorverbindung F2 (direkter Tracer)
- oder an die Ligandenstelle E1, die an den Kalibrier-Mikrokugeln verwendet wird (indirekter Tracer), und außerdem wird bei der Reaktion ein Antiligand-E1-Entwicklungstracer verwendet, der an die Fluorophorverbindung F2 gebunden ist (heterologe Kalibrierung).

2. Kalibrierverfahren nach Anspruch 1, **dadurch gekennzeichnet, dass** ein indirekter Entwicklungstracer in Form eines an eine Fluorophorverbindung F2 gebundenen Antiliganden E1 verwendet wird.

3. Kalibrierverfahren nach Anspruch 1 oder 2, **dadurch gekennzeichnet, dass** die erste Kategorie von Mikrokugeln mindestens zwei Unterkategorien von Mikrokugeln umfasst, wobei sich diese beiden Unterkategorien durch ihre Anzahl an Ligandenstellen E1 voneinander unterscheiden.

4. Kalibrierverfahren nach Anspruch 1, 2 oder 3, **dadurch gekennzeichnet, dass** die Kalibrierung gleichzeitig mit der Bestimmung auch des Messsignals eines Analyten unter Verwendung ein- und desselben Detektionssystems vorgenommen wird, und **dadurch gekennzeichnet, dass** die Fluoreszenzen an jeder Mikrokugel mit Hilfe eines Detcktionssystems nacheinander analysiert werden, so dass die Messungen an der Gesamtheit der Kalibrier-Mikrokugeln und an der Gesamtheit der Dosierungs-Mikokugeln, welche bei der Dosierung einer Probe zum Einsatz gelangen, durchgeführt werden, statistisch äquivalent sind.

5. Kalibrierverfahren nach einem der Ansprüche 1 bis 4, **dadurch gekennzeichnet, dass** die verschiedenen Reaktionen vom angewendeten Ligand/Antiligand-Typ von der Art Antigen - Antikörper sind.

6. Kalibrierverfahren nach einem der Ansprüche 1 bis 4, **dadurch gekennzeichnet, dass** die verschiedenen Reaktionen vom angewendete Ligand/Antiligand-Typ von der Art Nukleinsäure - komplementäre Nukleinsäure sind.

7. Kalibrierverfahren nach einem der Ansprüche 1 bis 4, **dadurch gekennzeichnet, dass** die verschiedenen Reaktionen vom angewendeten Ligand/Antiligand-Typ von der Art Enzym - Inhibitor sind.

8. Kalibrierverfahren nach einem der Ansprüche 1 bis 7, **dadurch gekennzeichnet, dass** die Kalibrier-Mikrokugeln und die Dosierungs-Mikrokugeln dieselbe Größe aufweisen.

9. Kalibrierverfahren nach einem der Ansprüche 3 bis 8, **dadurch gekennzeichnet, dass** der Parameter zur Unterscheidung der Unterkategorien von Kalibrierkugeln untereinander eine Fluoreszenzintensität ist.

10. Kalibrierverfahren nach einem der Ansprüche 1 bis 7 und 9, **dadurch gekennzeichnet, dass** die Parameter zur Unterscheidung der Kalibrier- und Messkugeln für jeden Analyten Kombinationen aus Intensität der Fluoreszenz F1 und Größe sind.

11. Dosierungs-Kit, bei dem das Kalibrierverfahren nach einem der Ansprüche 1 bis 10 zum Einsatz gelangt, umfassend
- Kalibrier-Mikrokugeln,
- Dosierungs-Mikrokugeln,
- einen Kalibriertracer,
- einen Dosierungstracer (der auch als Kalibriertracer für die Bedingung i) dient) für die Bedirigungen ii), iii) und iiii) wie in Anspruch 1 definiert und
- eine Korrespondenztafel Kalibriermikrokugeln - numerische Daten, die zur Interpretation eines Messsignals notwendig sind.

12. Dosierungs-Kit nach Anspruch 11, **dadurch gekennzeichnet, dass** er die Reagenzien umfasst, die einer der Bedingungen i), ii), iii) und iiii) wie in Anspruch 1 definiert entsprechen.

## Claims

1. A method of calibrating a system for assaying an analyte in a biological sample, using a specific ligand/anti-ligand type of interaction in a reaction schema of the "sandwich" or "competition" type, **characterized in that** the biological sample is reacted with a mixture of microspheres comprising:
- a first category of microspheres that can be **characterized by** their size, the fluorescence spectrum (F1) or by the intensity of fluorescence (F1), called calibration microspheres, these microspheres comprise an E1 ligand site, and with
- a tracer formed by an anti-ligand that is specific to the E1 ligand site and is complexed with an F2 fluorophor compound with a fluorescence different from that (F1) which can characterize the calibration microspheres and with
- a second category of microspheres, called analysis microspheres, different from the first category of microspheres either with respect to size, or fluorescence spectrum (F1) or intensity of fluorescence (F1), and which constitutes the medium of the analytical reaction, according to one of the following embodiments:
i) in the case where the analyte has an E1 site identical to that carried by the calibration microspheres, the said category is complexed with an anti-ligand that is specific to an E2 ligand site of the analyte, and an anti-ligand tracer E1 complexed with the F2 fluorophor compound is also used in the reaction (homologous calibration).
ii) said second category is complexed with an anti-ligand that is specific to an E2 ligand site of the analyte, and an anti-ligand specific to an E3 site of said analyte is also used in the reaction, this anti-ligand being complexed
- either with the same F2 fluorophor compound (direct tracer),
- or with the E1 ligand site used on the calibration microspheres (indirect tracer), and an Elanti-ligand detection tracer complexed with the F2 fluorophor compound is also used in the reaction (heterologous calibration),
iii) the said second category is complexed with an anti-ligand specific to an E2 ligand site of the analyte, and a ligand that is an analogue of the analyte carrying an E2 ligand site is also used in the reaction, this analogue being complexed
- either with the same F2 fluorophor compound (direct tracer),
- or with the E1 ligand site used on the calibration microspheres (indirect tracer), and an E1 anti-ligand detection tracer complexed with an F2 fluorophor compound (heterologous calibration) is also used in the reaction,
iiii) the said second category is complexed with an analyte or with an analogue of the analyte carrying an E2 ligand site, and an E2 anti-ligand is also used in the reaction, this anti-ligand being complexed
- either with the same F2 fluorophor compound (direct tracer),
- or with the E1 ligand site used on the calibration miscrospheres (indirect tracer), and an E1 anti-ligand detection tracer complexed with the F2 fluorophor compound (heterologous calibration) is also used in the reaction.

2. A method of calibration according to claim 1, **characterized in that** an indirect detection tracer is used which is an E1 anti-ligand complexed with an F2 fluorophor compound.

3. A method of calibration according to claim 1 or 2, **characterized in that** the first category of microspheres comprises at least two sub-categories of microspheres, these two sub-categories differing from one another in their quantity of E1 ligand sites.

4. A method of calibration according to claim 1, 2 or 3, **characterized in that** calibration is carried out simultaneously with the actual determination of the measurement signal of an analyte using an identical detection system and **characterized in that** the fluorescences are analysed on each microsphere successively, by a detection system, in such a way that the measurements carried out on all of the calibration microspheres and on all of the analysis microspheres employed during assay of a sample are statistically equivalent.

5. A method of calibration according to one of claims 1 to 4, **characterized in that** the various reactions of the ligand/anti-ligand type used are of an antigen-antibody nature.

6. A method of calibration according to one of claims 1 to 4, **characterized in that** the various reactions of the ligand-anti-ligand type used are of the nucleic acid-complementary nucleic acid nature.

7. A method of calibration according to one of claims 1 to 4, **characterized in that** the various reactions of the ligand-anti-ligand type used are of the enzyme-inhibitor nature.

8. A method of calibration according to one of claims 1 to 7, **characterized in that** the calibration microspheres and the analysis microspheres are of the same size.

9. A method of calibration according to one of claims 3 to 8, **characterized in that** the parameter for mutual discrimination of the sub-categories of calibration microspheres is an intensity of fluorescence.

10. A method of calibration according to one of claims 1 to 7 and 9, **characterized in that** the parameters for discrimination of the calibration and measurement microspheres for each analyte are combinations of intensity of F1 fluorescence and size.

11. An assay kit using the method of calibration as defined in one of claims 1 to 10 comprising
- calibration microspheres
- analysis microspheres
- a calibration tracer
- an analysis tracer (also serving as calibration tracer for embodiment i), for embodiments ii), iii), and iiii) as defined in claim 1 and
- a table of correspondence between calibration microspheres and numerical data, necessary for interpreting a measured signal.

12. An assay kit according to claim 11, **characterized in that** it comprises the reagents corresponding to one of the embodiments i), ii), iii) and iiii) as defined in claim 1.
